# EUROPÄISCHE PATENTSCHRIFT

(11) **EP 0 977 825 B1**
(45) Veröffentlichungstag und Bekanntmachung des Hinweises auf die Patenterteilung: **09.10.2002**
(21) Anmeldenummer: 98924121.1
(22) Anmeldetag: 15.04.1998
(51) Int. Cl.: C11D 3/00

(54) **FLÜSSIGE ENZYMZUBEREITUNG UND IHRE VERWENDUNG**
LIQUID ENZYME PREPARATION AND THE USE THEREOF
PREPARATION ENZYMATIQUE LIQUIDE ET SON UTILISATION

(30) Priorität: 24.04.1997 DE 19717329
(43) Veröffentlichungstag der Anmeldung: 09.02.2000
(73) Patentinhaber: Ecolab GmbH & Co. OHG, 40589 Düsseldorf (DE)
(72) Erfinder: BRAGULLA, Siegfried, D-40789 Monheim (DE); POTTHOFF, Andreas, D-40589 Düsseldorf (DE); SERVE, Wilfried, D-51371 Leverkusen (DE)
(86) Internationale Anmeldenummer: EP9802200
(87) Internationale Veröffentlichungsnummer: WO98047993

(56) Entgegenhaltungen:
- EP-A- 0 508 381
- WO-A-96/06910
- GB-A- 1 354 761
- US-I5- B 458 819
- DATABASE WPI Section Ch, Derwent Publications Ltd., London, GB; Class A97, AN 73-62619U XP002077577 & JP 48 049984 A (SUNSTAR DENTIFRICE CO LTD)

## Beschreibung

Die im folgenden beschriebene Erfindung liegt auf dem Gebiet der enzymatischen Reinigung von harten Oberflächen. Sie betrifft insbesondere eine flüssige stabilisierte Enzymzubereitung und deren Verwendung zur Reinigung harter Oberflächen, vor allem im Molkereibetrieb.

Auf Oberflächen von Behältern und Leitungen, die bei der Milchgewinnung und Milchverarbeitung mit Milch oder Milchprodukten in Berührung kommen, bilden sich Ablagerungen, die Proteine und Fette enthalten und die nur sehr schwer zu entfernen sind. In besonders starkem Maße finden sich derartige Ablagerungen in Melkmaschinen, Milchsammelwagen, Tanks, Rohrleitungen und Erhitzern. Wegen der Hartnäckigkeit der Anschmutzungen verwendet man zur Reinigung derartiger Gerätschaften im allgemeinen stark alkalische und teilweise zusätzlich stark saure Reiniger, die wegen ihrer stark ätzenden Eigenschaften mit großer Vorsicht gehandhabt werden müssen. An empfindlichen Materialien muß darüber hinaus beim Einsatz derartiger Reiniger mit Korrosion gerechnet werden, wenn nicht besondere Vorsichtsmaßnahmen ergriffen werden. Vor der Abgabe der Reiniger an die Kanalisation ist eine Neutralisation unumgänglich.

Wegen der Schwierigkeiten beim Einsatz hoch alkalischer bzw. stark saurer Reinigerlösungen hat es nicht an Versuchen gefehlt, Reinigungslösungen zu entwickeln, die bei annähernd neutralem pH-Wert eine ähnliche Reinigungsleistung wie die stark alkalischen bzw. stark sauren Reiniger aufweisen. Hier wurde vor allem der Einsatz von Enzymen, insbesondere von Proteasen vorgeschlagen. In der Literatur sind zahlreiche Veröffentlichungen zu diesem Thema erschienen, von denen an dieser Stelle nur die internationale Patentanmeldung WO 96/6910 erwähnt werden soll, in der sich weitere Literaturzitate befinden. Grundsätzlich ist beim Einsatz von Proteasen eine ausreichende Reinigung auch im Neutralbereich möglich, doch ist der Einsatz dieser Enzyme mit anderen Schwierigkeiten verknüpft. So werden insbesondere in der gewerblichen Reinigung flüssige Konzentrate zur Herstellung der eigentlichen Reinigungslösung bevorzugt. Flüssige Enzymkonzentrate verlieren aber bei Lagerung rasch ihre Wirksamkeit, da die enthaltenen Enzyme denaturieren oder auf andere Weise ihre Aktivität verlieren. Es sind daher zahlreiche Versuche unternommen worden, flüssige Enzymzubereitungen durch den Zusatz sogenannter Stabilisatoren besser lagerfähig zu machen. Weite Verbreitung hat beispielsweise der Zusatz von Borsäure oder Boraten und von löslichem Kalziumsalzen und auch der Zusatz von mehrwertigen Alkoholen gefunden. Eine allseits befriedigende Lösung des Problems ist aber bisher mit diesen Stabilisatoren nicht gelungen.

Die britische Patentveröffentlichung GB-A-1 354 761 beschreibt ein wässriges Wasch-und Reinigungskonzentrat, das einen pH- Wert von 5.0 bis 10.0 aufweist und mit Wasser verdünnt werden kann. Es enthält stabilisierte Enzyme (z.B. Protease), Di-bis Hexahydroxyalkane, Komplexbildner sowie alkalisch reagierende Salze. Desweiteren ist ein Bis-(3 aminopropyl) methylamin oder polyfunktionales Alkylamin mit maximal 8 Kohlenstoffatomen pro Substituent und mindestens einem weiteren einfachen Stickestoff als Stabilisierungsreagenz enthalten.

Auch der im folgenden beschriebenen Erfindung liegt die Aufgabe zugrunde, die Stabilität flüssiger Enzymzubereitungen zu erhöhen. Eine weitere Aufgabe ist, derartige Enzymzubereitungen zu entwickeln, die sich zur Reinigung harter Oberflächen, insbesondere im Bereich der Milchwirtschaft, eignen. Weiterhin sollen diese Enzymzubereitungen geeignet sein, wiederverwendbare Reinigungslösungen herzustellen, wie sie vor allem im Bereich der gewerblichen Reinigung benötigt werden.

Überraschenderweise wurde gefunden, daß eine Lösung der vorgenannten Aufgaben durch den Zusatz bestimmter basischer Stickstoffverbindungen zu den flüssigen Enzymzubereitungen möglich ist.

Gegenstand der Erfindung ist daher eine flüssige stabilisierte Enzymzubereitung, die ein reinigungswirksames Enzym , ein Lösungsmittel aus der Gruppe Ethylenglykol, Propylenglykol, Glycerin und deren Mischungen sowie ein Stabilisierungsmittel aus der Gruppe Polyhexamethylenbiguanid, N,N-Bis(3-aminopropyl)dodecylamin, deren Salze und Mischungen dieser Verbindungen enthält. Vorzugsweise enthält die Enzymzubereitung außerdem Wasser und gegebenenfalls weitere Hilfsstoffe, insbesondere aus der Gruppe Tenside, Lösungsvermittler und deren Mischungen. Weitere Gegenstände der Erfindung sind die Verwendung der Enzymzubereitung zur Herstellung einer Reinigungslösung für die Reinigung von Molkereigeräten sowie ein Verfahren zur Reinigung von Molkereigeräten mit Hilfe derartiger Reinigungslösungen im annähernd neutralen bis schwach alkalischen Bereich.

Bei den zur Stabilisierung der flüssigen Enzymzubereitung eingesetzten basischen Stickstoffverbindungen handelt es sich um Substanzen, die bisher naherzu ausschließlich wegen ihrer antimikrobiellen Wirksamkeit in Desinfektionsmitteln Verwendung gefunden haben. So wird Polyhexamethylenbiguanid-Hydrochlorid unter den Namen Lonzabac BG bzw. Vantocil IB für diesen Zweck von den Firmen Lonza bzw. Zeneca Biozides angeboten. N,N-Bis(3-aminopropyl)dodecylamin wird unter der Bezeichnung Lonzabac®-12.30 bzw. unter der Bezeichnung Bardac® 21 von der Firma Lonza für diesen Zweck angeboten. Daß diese antimikrobiellen Wirkstoffe eine stabilisierende Wirkung auf flüssige Enzymzubereitungen haben, war nicht bekannt. Besonders vorteilhaft ist, daß bereits sehr geringe Mengen an diesen basischen Stickstoffverbindungen ausreichen, um die flüssige Enzymzubereitung zu stabilisieren. Vorzugsweise werden sie in Mengen zwischen 0,5 und 5 Gew.-%, insbesondere in Mengen zwischen 1 und 3 Gew.-%, bezogen auf die flüssige Enzymzubereitung als ganzes, eingesetzt, doch kann es in Sonderfällen auch zweckmäßig sein, die Stabilisierungsmittel in Mengen außerhalb dieser Konzentrationsbereiche anzuwenden. Die oben angegebenen Handelsformen der beiden Stabilisierungsmittel als Hydrochlorid bzw. freie Base sind die bevorzugten Einsatzformen, doch ist es selbstverständlich auch möglich, andere Salze der basischen Stickstoffverbindungen zu verwenden, wenn das im Einzelfall zweckmäßig sein sollte. Geeignet sind außer den basischen Stickstoffverbindungen selbst alle in der Enzymzubereitung löslichen Salze mit anorganischen oder organischen Säuren.

Als Lösungsmittel enthalten die flüssigen Enzymzubereitungen wenigstens eine Substanz aus der Gruppe Ethylenglykol, Propylenglykol und Glycerin, wobei diese Lösungsmittel allein vorliegen können, vorzugsweise aber zusammen mit Wasser als weiterem Lösungsmittel verwendet werden. Der Gehalt an Lösungsmitteln aus der Gruppe Ethylenglykol, Propylenglykol, Glycerin und deren Mischungen beträgt in den Enzymzubereitungen vorzugsweise 10 bis 70 Gew.-%, insbesondere 30 bis 50 Gew.-% bezogen auf die Zubereitung als ganzes. Sofern zusätzlich Wasser als Lösungsmittel verwendet wird, beträgt dessen Gehalt vorzugsweise 5 bis 40 Gew.-%, insbesondere 10 bis 30 Gew.-%, wiederum bezogen auf die flüssige Enzymzubereitung als ganzes. Da Kalziumsalze in der Regel einen stabilisierenden Effekt auf Enzymlösungen haben, wird für die Enzymzubereitungen vorzugsweise Leitungswasser verwendet.

Auch wenn sich die stabilisierende Wirkung der obengenannten stickstoffhaltigen Basen auf flüssige Zubereitungen beliebiger Enzyme erstreckt, wird sie erfindungsgemäß vorzugsweise für die Stabilisierung von Lösungen reinigungswirksamer Enzyme ausgenutzt, d.h. für solche Enzyme, die in irgendeiner Weise bei der Reinigung von harten Oberflächen oder Textilien von Nutzen sind. Die erfindungsgemäß stabilisierten Enzymzubereitungen enthalten daher vorzugsweise wenigstens ein Enzym aus der Gruppe Proteasen (Peptidasen), Amylasen, Cellulasen, Glykosidasen, Lipasen und Oxidoreduktasen. Die erfindungsgemäßen Zubereitungen können mehrere Enzyme auch aus verschiedenen Klassen enthalten, sofern keine störenden Wechselwirkungen zwischen den Enzymen auftreten. Für den besonders bevorzugten Anwendungszweck der erfindungsgemäßen Zubereitungen, die Reinigung von festen Oberflächen im Bereich der Milchverarbeitung, enthalten die Zubereitungen wenigstens eine Protease, insbesondere eine Protease allein oder im Gemisch mit weiteren Proteasen.

Die Menge an Enzym wird in den erfindungsgemäßen Zubereitungen danach gewählt, welchem Einsatzzweck die Zubereitungen dienen sollen. Dementsprechend kann die Menge an Enzymen in sehr weiten Grenzen frei gewählt werden. Die vorteilhafte stabilisierende Wirkung der basischen Stickstoffverbindungen tritt bei allen Enzymkonzentrationen auf. Selbstverständlich richtet sich die Enzymkonzentration in den Zubereitungen auch nach der spezifischen Aktivität des jeweils eingesetzten Enzyms. Soweit deshalb im folgenden Mengenangaben für die Enzyme gemacht werden, sind diese nur als grobe Richtgrößen anzusehen, die im Einzelfall bei Bedarf ohne weiteres über oder unterschritten werden können. Die Konzentrationsangaben gelten für reines Enzym und beziehen die von den Herstellern den Enzympräparaten häufig beigemischten Verschnittmittel, Hilfsmittel oder Lösungsmittel nicht mit ein. Andererseits werden die erfindungsgemäßen Enzymzubereitungen üblicherweise aus den bereits vorkonfektionierten Enzymen, wie sie von den Herstellern angeboten werden, hergestellt, so daß die Hilfs- und Verschnittmittel automatisch zu Bestandteilen der erfindungsgemäßen Zubereitungen werden. Vorzugsweise wird bei der Herstellung der erfindungsgemäßen Zubereitung von hochkonzentrierten flüssigen Enzympräparaten ausgegangen, die von verschiedenen Herstellern angeboten werden. So sind beispielsweise flüssige Proteasen unter den Bezeichnungen Alkalase®, Savinase®, Purafect® und Maxatase® von Firmen wie Genencor und Novo erhältlich. Vorzugsweise werden Enzyme eingesetzt, die ihr Wirkungsoptimum in der Nähe des pH-Wertes haben, bei dem die Enzymzubereitungen zur Reinigung verwendet werden. Vorzugsweise enthalten die erfindungsgemäßen Enzymzubereitungen 1 bis 15 Gew.-%, insbesondere 2 bis 8 Gew.-% an reinigungswirksamem Enzym. Im Falle von Proteasen soll dies vorzugsweise 5 bis 100 Anson-Einheiten pro 100 g der Zubereitung entsprechen.

Neben den bereits genannten Bestandteilen können die erfindungsgemäßen Enzymzubereitungen weitere Hilfsstoffe enthalten, wenn das im Einzelfalle zweckdienlich ist. In erster Linie sind hier Tenside zur Verstärkung der Reinigungswirkung und, im Falle wasserhaltiger Zubereitungen, Lösungsvermittler (Hydrotrope) zu nennen. Beispiele für weitere Hilfs- und Zusatzstoffe sind andere übliche Enzymstabilisatoren, wie lösliche Kalziumsalze und Borate, Verdickungsmittel, Farbstoffe, Antioxidantien, Schauminhibitoren, Konservierungsmittel und Puffersubstanzen, Bei der Wahl sämtlicher Hilfs- und Zusatzstoffe ist darauf zu achten, daß keine störenden Wechselwirkungen mit den übrigen Bestandteilen der erfindungsgemäßen Zubereitungen auftreten. So können prinzipiell Tenside aus allen bekannten Klassen Verwendung finden, doch werden nichtionische, kationische und amphotere Tenside besonders bevorzugt, von denen wiederum die nichtionischen Tenside die größte Bedeutung besitzen.

Geeignete nichtionische Tenside sind insbesondere die Additionsprodukte aus langkettigen Alkoholen, Alkylphenolen, Amiden und Carbonsäuren mit Ethylenoxid (EO) gegebenenfalls zusammen mit Propylenoxid (PO). Hierzu zählen beispielsweise die Additionsprodukte von langkettigen primären oder sekundären Alkoholen mit 12 bis 18 C-Atomen in der Kette, insbesondere von Fettalkoholen und Oxoalkoholen dieser Kettenlänge und die Additionsprodukte aus Ethylenoxid und Fettsäuren mit 12 bis 18 C-Atomen in der Kette, mit vorzugsweise 2 bis 6 Mol Ethylenoxid. Besonders bevorzugt werden die gemischten Additionsprodukte aus Ethylen- und Propylenoxid und Fettalkoholen mit 12 bis 18 C-Atomen, insbesondere solche, die 2 Mol EO und 4 Mol PO im Molekül enthalten. Beispiele geeigneter nichtionischer Tenside sind etwa die unter den Bezeichnungen Dehypon® LS24, Dehypon® LS54, Emulgin® 05, Dehydol® LT8, Dehydol® LT6 und Dehydol® LS6 von der Firma Henkel angebotenen Fettalkoholalkoxylate.

Weitere geeignete nichtionische Tenside sind die Ester aus Fettsäuren mit 6 bis 12 C-Atomen und Polyolen, insbesondere Kohlenhydraten, z.B. Glukose. Sofern nichtionische Tenside in den erfindungsgemäßen Enzymzubereitungen enthalten sind, beträgt ihr Anteil vorzugsweise 5 bis 50 Gew.-%, insbesondere 20 bis 40 Gew.-%, bezogen auf die Zubereitung als ganzes.

Als kationische Tenside eignen sich insbesondere aliphatische oder heterocyclische quartäre Ammoniumverbindungen und quartäre Phosphoniumverbindungen, die am quartären Zentrum wenigstens einen langkettigen C₈ - C₁₈-Alkylrest aufweisen. Beispiele derartiger kationischer Tenside sind etwa Kokosalkyl-benzyl-dimethylammoniumchlorid, Dioctyl-dimethylammoniumchlorid und Tributyltetradecylphosphoniumchlorid.

Als amphotere Tenside eignen sich insbesondere C₈ - C₁₈-Fettsäureamidderivate mit Betainstruktur, insbesondere Derivate des Glycins, beispielsweise Kokosalkyldimethylammoniumbetain.

Beispiele geeigneter Lösungsvermittler sind Cumolsulfonat, Xylolsulfonat und Octylsulfonat, doch können selbstverständlich auch hier andere übliche Lösungsvermittler eingesetzt werden. Der Gehalt an Lösungsvermittler wird nach Bedarf gewählt und beträgt vorzugsweise 1 bis 10 Gew.-%, insbesondere 2 bis 5 Gew.-%, bezogen auf die Zubereitung als ganzes.

Die erfindungsgemäßen Enzymzubereitungen werden für die Anwendung zur Reinigung üblicherweise nicht als solche eingesetzt, sondern bilden das Ausgangsmaterial für die Herstellung der eigentlichen Reinigungslösung. Im einfachsten Falle wird die Reinigungslösung durch Verdünnen der Enzymzubereitung mit Wasser hergestellt, doch werden in vielen Fällen der Reinigungslösung noch weitere Bestandteile hinzugefügt. Bei der bevorzugten Verwendung der Enzymzubereitung zur Reinigung von Molkereigeräten werden die Enzymzubereitungen mit Wasser verdünnt, wobei vorzugsweise ein Verhältnis von etwa 1 : 5000 bis 1 : 500, insbesondere 1 : 3000 bis 1 : 1000 eingehalten wird. In einer besonders bevorzugten Ausführungsform werden dieser Reinigungslösung für diesen Zweck weitere reinigungsaktive Substanzen, insbesondere aus der Gruppe der Komplexbildner, der Alkalien und der Tenside zugesetzt. Selbstverständlich ist auch der Zusatz weiterer Hilfsstoffe an dieser Stelle möglich, beispielsweise der Zusatz von Schauminhibitoren und Puffersubstanzen, wie etwa löslichen Carbonaten oder Silikaten.

In einem besonderen Reinigungsverfahren für Molkereigeräte, das ebenfalls Gegenstand der vorliegenden Erfindung ist, wird die Reinigungslösung durch Verdünnen einer erfindungsgemäßen Enzymzubereitung im oben angegebenen Verhältnis mit Wasser und weiteren Zusatz eines Pufferkonzentrats hergestellt. Von diesem Pufferkonzentrat werden 0,01 bis 0,2 Gew.-%, vorzugsweise 0,03 bis 0,1 Gew.-%, bezogen auf die verdünnte Reinigungslösung als ganzes, eingesetzt mit dem Ziel, diese Reinigungslösung auf einen pH-Wert zwischen 7 und 10, vorzugsweise zwischen 8 und 9,5 einzustellen. Je nach Ausgangs-pH-Wert der Enzymzubereitung kann dieses Pufferkonzentrat sauer oder alkalisch reagierende Substanzen enthalten, z.B. Zitronensäure, Natriumcarbonat oder Natriumhydrogencarbonat.

Ein für die Einstellung eines alkalischen pH-Wertes besonders bevorzugtes Pufferkonzentrat hat folgende Zusammensetzung:
- 3 bis 20 Gew.-%,: vorzugsweise 5 bis 10 Gew.-% Komplexbildner aus der Gruppe der Polyphosphonate, Polyacrylate und deren Mischungen,
- 3 bis 20 Gew.-%,: vorzugsweise 5 bis 10 Gew.-% Alkalihydroxid aus der Gruppe Natriumhydroxid, Kaliumhydroxid und deren Mischungen,
- 5 bis 30 Gew.-%,: vorzugsweise 10 bis 20 Gew..-% Alkalicarbonat aus der Gruppe Natriumcarbonat, Kaliumcarbonat und deren Mischungen und
- 20 bis 80 Gew.-%,: vorzugsweise 40 bis 60 Gew.-% Wasser

Die Reinigung der Molkereigeräte mit der auf diese Weise hergestellten Reinigungslösung kann manuell erfolgen, wird vorzugsweise aber mehr oder weniger maschinell durchgeführt. Üblicherweise werden die Geräte zunächst mit gegebenenfalls erwärmten Wasser vorgespült, bevor die eigentliche Reinigung mit der erfindungsgemäß hergestellten Reinigungslösung durchgeführt wird. Die Reinigung kann bei Raumtemperatur vorgenommen werden, wird aber vor allem bei maschineller Reinigung vorzugsweise bei erhöhten Temperaturen, insbesondere im Bereich bis 70 °C durchgeführt. Besonders bevorzugt werden Temperaturen im Bereich zwischen 40 und 60 °C, insbesondere zwischen 50 und 60 °C. Die Reinigung kann durch Anwendung von Mechanik unterstützt werden, doch reicht vor allem bei höheren Temperaturen die Bewegung der Reinigungslösung für eine zufriedenstellende Reinigung aus. Bei geschlossenen Anlagen wird die Reinigungslösung im Kreislauf umgepumpt und gegebenenfalls, etwa im Inneren von Tanks, durch Sprühköpfe oder ähnliche Vorrichtungen auf die zu reinigenden Oberflächen verteilt. Die Umwälzung der Reinigungslösung wird für eine ausreichende Zeit, beispielsweise für 5 bis 30 Minuten beibehalten, bis der erwünschte Reinigungsgrad erreicht ist. Dieses Reinigungsverfahren wird auch als "Cleaning-in-place" bezeichnet (CIP). Nach dem Abpumpen der Reinigungslösung werden die Geräte üblicherweise mit Frischwasser gespült und, sofern nötig, nach Entfernung des Frischwassers auch noch mit einer Desinfektionslösung und nochmals mit Frischwasser behandelt.

Besonders vorteilhaft ist, daß die mit der erfindungsgemäßen Enzymzubereitung hergestellten Reinigungslösungen aufgrund ihres Gehalts an den mikrobizid wirkenden basischen Stickstoffverbindungen nicht dem mikrobiellen Verderb unterliegen und deshalb für weitere Reinigungsvorgänge aufbewahrt werden können. Sie werden dazu in Lagertanks aufbewahrt und nur jeweils um die Menge an Lösung ergänzt, die beim Reinigungsverfahren in den Geräten verblieben und mit dem Nachspülwasser verlorengegangen ist. Da auf diese Weise bei jedem Reinigungsvorgang nur wenige Prozente der Reinigungslösung ins Abwasser gelangen, ist die Abwasserbelastung gegenüber herkömmlichen Verfahren, bei denen jedesmal die gesamte Reinigungslösung verworfen werden muß, außerordentlich gering. Gegenüber den wiederverwendbaren, hochalkalischen Reinigern sind das Fehlen von Phosphat in der Reinigungslösung und die geringe Korrosion als Vorteile zu werten.

### Beispiele

### 1. Stabilisierte flüssige Enzymzubereitungen

Durch Vermischen der einzelnen Bestandteile wurden drei flüssige Enzymzubereitungen 1, 2 und 3 hergestellt, von denen die Zubereitungen 2 und 3 erfindungsgemäß sind, während die Zubereitung 1 keine stabilisierende Stickstoffverbindung enthält. Die Gehalte an Einzelkomponenten sind für die drei Zubereitungen in Tabelle 1 in Gewichtsprozent angegeben.

Die fertig hergestellten Zubereitungen wurden zur Prüfung der Lagerstabilität einem verschärften Lagertest bei 60 °C unterworfen. Die Zubereitungen wurden dazu in verschlossenen Glasgefäßen in einem Wärmeschrank gelagert. Nach 20, 40 und 60 Minuten wurden Proben entnommen, in denen der Enzymgehalt mit einer Standardmethode bestimmt wurde. Die Ergebnisse sind ebenfalls in Tabelle 1 in Form von Prozentangaben, bezogen auf einen Anfangswert von 100 %, aufgeführt. Die stabilisierende Wirkung der stickstoffhaltigen Verbindungen ist ohne weiteres erkennbar.

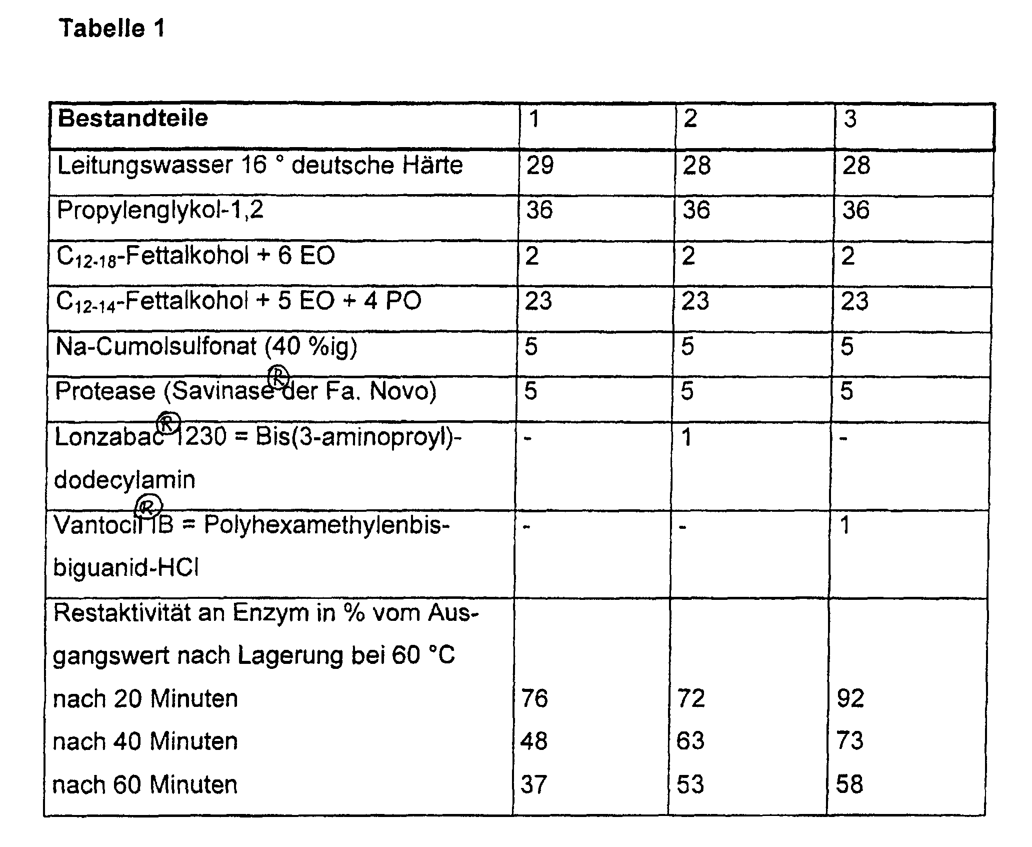

### 2. Reinigungswirkung der enzymatischen Reinigungslösungen

Die Reinigungswirkung wurde an künstlich angeschmutzten Edelstahlplättchen im Vergleich zu einem herkömmlichen hochalkalischen Reiniger geprüft. Die Herstellung der Standardanschmutzung erfolgte auf folgende Weise:
Edelstahlplättchen der Größe 5 x 10 cm wurden mit 0,25 g Kondensmilch (Fettgehalt 10 %) benetzt und anschließend getrocknet. Die auf diese Weise angeschmutzten Edelstahlplättchen wurden mittels einer Hubmechanik 10 mal in die jeweilige auf 50 °C erhitzte Reinigungslösung (2000 ml) eingetaucht und anschließend mit destilliertem Wasser abgespült und getrocknet. Die Reinigungswirkung wurde gravimetrisch ermittelt, indem aus dem Gewicht der unbehandelten, der angeschmutzten und der gereinigten Edelstahlplättchen die Menge an abgelöster Anschmutzung berechnet wurde.

Als Vergleichsprodukt diente ein Reinigerkonzentrat folgender Zusammensetzung:
35 Gew.-% Natriumhypochlorid-Lösung (12 %ig)
15 Gew.-% Natronlauge (50 %ig)
5 Gew.-% K₅P₃O₁₀
40 Gew.-% destilliertes Wasser

Zur Herstellung der Reinigungslösung wurde dieses Konzentrat im Verhältnis 1 : 100 mit Leitungswasser verdünnt; die Reinigungslösung hatte einen pH-Wert von 12,3.

Als erfindungsgemäß wurde eine flüssige Enzymzubereitung folgender Zusammensetzung geprüft:
10 Gew.-% flüssiges Proteasepräparat (Savinase® 16.0 LEX, Firma Novo)
40 Gew.-% Propylenglykol-1,2
20 Gew.-% C₁₂₋₁₄-Fettalkohol + 4 EO + 5 PO
5 Gew.-% Polyhexamethylenbiguanid-Hydrochlorid
25 Gew.-% Wasser

Zur Herstellung der Reinigungslösung wurde diese Zubereitung mit Wasser im Verhältnis 1 : 1000 verdünnt. Durch Zusatz von Kaliumcarbonat wurde der pH-Wert der verdünnten Lösung auf 8,5 eingestellt.

Mit dem herkömmlichen Reiniger wurde mit der 1 %igen Lösung eine Reinigungswirkung von 99,3 % bei 50 °C erreicht. Dagegen wurde bereits mit einer 0,1 %igen Lösung der erfindungsgemäßen Enzymzubereitung eine Reinigungswirkung von 99,9 % bei 50 °C erreicht.

### 3. Verkeimung der Reinigungslösung

Zur Prüfung, ob die verdünnten Reinigerlösungen, die aus den erfindungsgemäßen Enzymzubereitungen enthalten werden, längere Zeit gegen mikrobiologischen Befall beständig sind und deshalb gelagert werden können, wurden derartige Lösungen mit einem Keimgemisch geimpft und der Keimgehalt dann nach 21 Tagen Lagerung bei 25 °C überprüft. Die Zusammensetzung der Zubereitungen, aus denen die Reinigungslösungen durch Verdünnen mit Wasser im Verhältnis 1 : 2000 hergestellt wurden, sind in Tabelle 2 in Gewichtsprozent wiedergegeben. Den fertigen Reinigungslösungen wurden 0,75 Gew.-% Rohmilch zugefügt, um die Belastung mit organischem Material zu simulieren. Pro Liter Reinigungslösung wurden dann 1 ml eines Bakterien- und Pilzgemisches zugesetzt, das folgende Keime enthielt:

| **Bakteriengemisch** (3 x 10⁸ /ml) aus: | |
|---|---|
| Staphylococcus aureus | ATCC 6538 |
| Enerococcus faecium | ATCC 6057 |
| Escherichia coli | ATCC 11229 |
| Pseudomonas aeruginosa | ATCC 15442 |
| Enterobacter aerogenes | DSM 30053 |

| **Pilzgemisch** (15 x 10⁷/ml) aus: | |
|---|---|
| Candida albicans | ATCC 10231 |
| Aspergillus niger | ATCC 6275 |
| Penicilium rubrum | CMI 113729 |
| Trichoderma viride | BAM T 21 |

Die nach Lagerung wiedergefundene Keimzahl (pro ml Reinigungslösung) ist ebenfalls in Tabelle 2 angegeben. Die keimhemmende Wirkung in den erfindungsgemäß hergestellten Reinigungslösungen (aus 5 und 6) ist ohne weiteres zu erkennen.

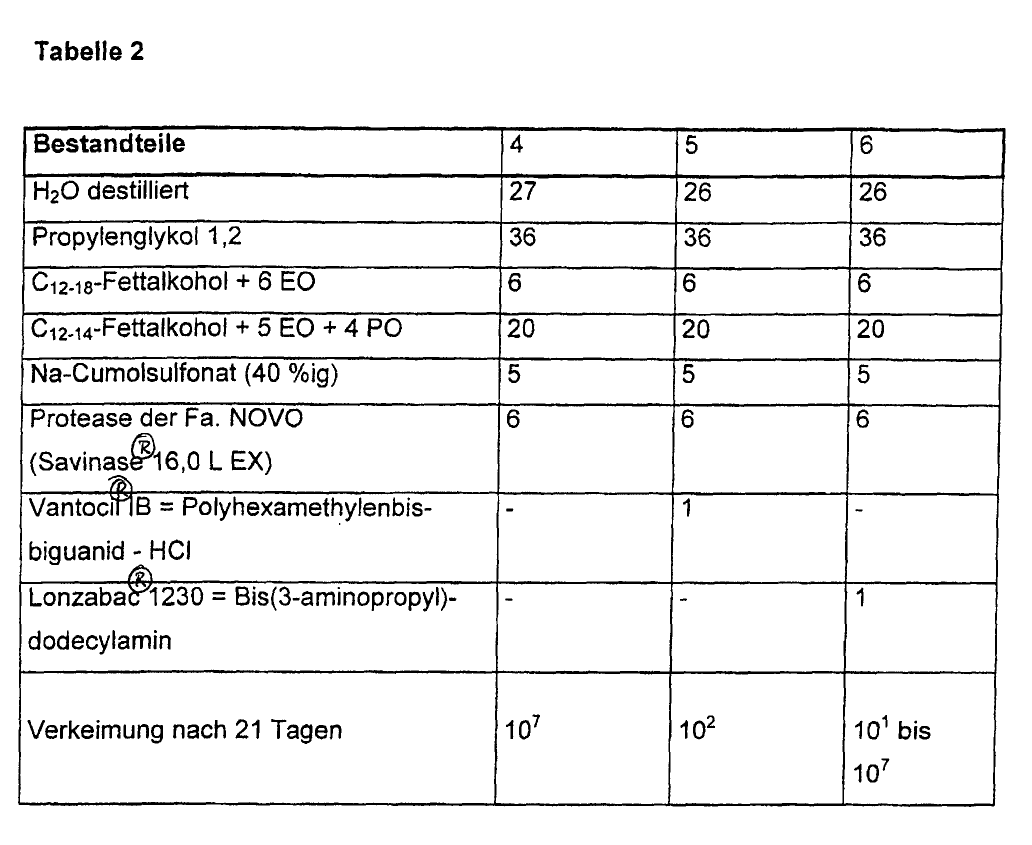

## Patentansprüche

1. Flüssige stabilisierte Enzymzubereitung, enthaltend ein reinigungswirksames Enzym, ein Lösungsmittel sowie eine basische Stickstoffverbindung als Stabilisierungsmittel, **dadurch gekennzeichnet, daß** das Lösungsmittel aus der Gruppe Ethylenglykol, Propylenglykol. Glycerin und deren Mischungen sowie die basische Stickstoffverbindung aus der Gruppe Polyhexamethylenbiguanid, N,N-Bis(3-aminopropyl)dodecylamin, deren Salze und Mischungen dieser Verbindungen ausgewählt ist.

2. Enzymzubereitung nach Anspruch 1, enthaltend wenigstens ein Enzym aus der Gruppe Proteasen, Lipasen und Amylasen, insbesondere wenigstens eine Protease.

3. Enzymzubereitung nach einem der Ansprüche 1 oder 2, die mindestens einen weiteren Hilfsstoff aus der Gruppe Tenside, Lösungsvermittler und deren Mischungen enthält.

4. Enzymzubereitung nach Anspruch 3 enthaltend nichtionische Tenside aus der Gruppe der Additionsprodukte aus Ethylenoxid und gegebenenfalls Propylenoxid und langkettigen Alkoholen mit 12 bis 18 C-Atomen in der Kette.

5. Enzymzubereitung nach einem der Ansprüche 1 bis 4, enthaltend:
10 bis 70 Gew.-%, vorzugsweise 30 bis 50 Gew.-% Lösungsmittel aus der Gruppe Ethylenglykol, Propylenglykol, Glycerin und deren Mischungen
0,5 bis 5 Gew.-%, vorzugsweise 1 bis 3 Gew.-% Stabilisierungsmittel aus der Gruppe Polyhexamethylenbiguanid, N,N-Bis(3-aminopropyl)dodecylamin, deren Salze und Mischungen dieser Verbindungen
1 bis 15 Gew.-%, vorzugsweise 2 bis 8 Gew.-% reinigungswirksames Enzym
10 bis 80 Gew.-% an weiteren Hilfsstoffen und Wasser

6. Enzymzubereitung nach einem der Ansprüche 1 bis 5, enthaltend 5 bis 40, insbesondere 10 bis 30 Gew.-% Wasser.

7. Enzymzubereitung nach einem der Ansprüche 1 bis 6, enthaltend 5 bis 50, insbesondere 20 bis 40 Gew.-% nichtionische Tenside aus der Gruppe der Additionsprodukte von Ethylenoxid und gegebenenfalls Propylenoxid mit Fettalkoholen.

8. Verwendung einer Enzymzubereitung nach einem der Ansprüche 1 bis 7 zur Herstellung einer Reinigungslösung für die Reinigung von Molkereigeräten.

9. Verfahren zur Reinigung von Molkereigeräten mit Hilfe einer Reinigungslösung, die durch Verdünnen einer Enzymzubereitung gemäß einem der Ansprüche 1 bis 7 mit Wasser im Verhältnis 1 : 5000 beis 1 : 500, vorzugsweise im Verhältnis 1: 3000 bis 1 : 1000 und nachfolgende Zugabe von 0,01 bis 0,2 Gew.-%, vorzugsweise 0,03 bis 0,1 Gew.-%, bezogen auf die Reinigungslösung als ganzes, eines Pufferkonzentrats zur Einstellung eines pH-Werts zwischen 7 und 10 hergestellt wird.

10. Verfahren nach Anspruch 9, bei dem das Pufferkonzentrat folgende Zusammensetzung hat:
3 bis 20 Gew.-%, vorzugsweise 5 bis 10 Gew.-% Komplexbildner aus der Gruppe der Polyphosphonate, Polyacrylate und deren Mischungen
3 bis 20 Gew.-%, vorzugsweise 5 bis 10 Gew.-% Alkalihydroxid aus der Gruppe Natriumhydroxid, Kaliumhydroxid und deren Mischungen
5 bis 30 Gew.-%, vorzugsweise 10 bis 20 Gew..-% Alkalicarbonat aus der Gruppe Natriumcarbonat, Kaliumcarbonat und deren Mischungen
20 bis 80 Gew.-%, vorzugsweise 40 bis 60 Gew.-% Wasser

11. Verfahren nach einem der Ansprüche 9 oder 10, bei dem die Reinigungslösung nach erfolgter Reinigung wieder aufgefangen und für spätere Reinigungsgänge aufbewahrt wird.

## Claims

1. A liquid stabilized enzyme preparation containing a detersive enzyme, a solvent and a basic nitrogen compound as stabilizer, **characterized in that** the solvent is selected from the group consisting of ethylene glycol, propylene glycol, glycerol and mixtures thereof and the basic nitrogen compound is selected from the group consisting of polyhexamethylene biguanide, N,N-bis-(3-aminopropyl)-dodecyl amine, salts thereof and mixtures of these compounds.

2. An enzyme preparation as claimed in claim 1 containing at least one enzyme from the group of proteases, lipases and amylases, more particularly at least one protease.

3. An enzyme preparation as claimed in claim 1 or 2 containing at least one other auxiliary from the group consisting of surfactants, solubilizers and mixtures thereof.

4. An enzyme preparation as claimed in claim 3 containing nonionic surfactants from the group consisting of addition products of ethylene oxide and optionally propylene oxide with long-chain alcohols containing 12 to 18 carbon atoms in the chain.

5. An enzyme preparation as claimed in any of claims 1 to 4 containing:
10 to 70% by weight and preferably 30 to 50% by weight of solvent from the group consisting of ethylene glycol, propylene glycol, glycerol and mixtures thereof,
0.5 to 5% by weight and preferably 1 to 3% by weight of stabilizer from the group consisting of polyhexamethylene biguanide, N,N-bis-(3-aminopropyl)-dodecyl amine, salts thereof and mixtures of these compounds,
1 to 15% by weight and preferably 2 to 8% by weight of detersive enzyme,
10 to 80% by weight of other auxiliaries and water.

6. An enzyme preparation as claimed in any of claims 1 to 5 containing 5 to 40% by weight and more particularly 10 to 30% by weight of water.

7. An enzyme preparation as claimed in any of claims 1 to 6 containing 5 to 50% by weight and more particularly 20 to 40% by weight of nonionic surfactants from the group consisting of addition products of ethylene oxide and optionally propylene oxide with fatty alcohols.

8. The use of the enzyme preparation claimed in any of claims 1 to 7 for preparing a cleaning solution for cleaning dairy equipment.

9. A process for cleaning dairy equipment using a cleaning solution prepared by diluting the enzyme preparation claimed in any of claims 1 to 7 with water in a ratio of 1:5000 to 1:500 and preferably in a ratio of 1:3000 to 1:1000 and subsequently adding 0.01 to 0.2% by weight and preferably 0.03 to 0.1% by weight, based on the cleaning solution as a whole, of a buffer concentrate for adjusting a pH value of 7 to 10.

10. A process as claimed in claim 9 in which the buffer concentrate has the following composition:
3 to 20% by weight and preferably 5 to 10% by weight of complexing agent from the group of polyphosphates, polyacrylates and mixtures thereof,
3 to 20% by weight and preferably 5 to 10% by weight of alkali metal hydroxide from the group consisting of sodium hydroxide, potassium hydroxide and mixtures thereof,
5 to 30% by weight and preferably 10 to 20% by weight of alkali metal carbonate from the group consisting of sodium carbonate, potassium carbonate and mixtures thereof and
20 to 80% by weight and preferably 40 to 60% by weight of water.

11. A process as claimed in claim 9 or 10 in which the cleaning solution is collected after cleaning and stored for subsequent cleaning operations.

## Revendications

1. Préparation liquide d'enzyme stabilisée, contenant une enzyme active pour le nettoyage, un solvant ainsi qu'un composé azoté basique en tant que stabilisant,
**caractérisée en ce que**
le solvant est choisi dans le groupe constitué par l'éthylèneglycol, le propylèneglycol, le glycérol et des mélanges de ceux-ci, et le composé azoté basique est choisi dans le groupe constitué par le polyhexaméthylênebiguanide, la N,N-bis(3-aminopropyl)dodécylamine, leurs sels et des mélanges de ces composés.

2. Préparation d'enzyme selon la revendication 1,
contenant au moins une enzyme choisie dans le groupe des protéases, lipases et amylases, en particulier au moins une protéase.

3. Préparation d'enzyme selon la revendication 1 ou 2,
qui contient au moins un autre adjuvant choisi dans le groupe des tensioactifs, des tiers-solvants et des mélanges de ceux-ci.

4. Préparation d'enzyme selon la revendication 3,
contenant des tensioactifs non ioniques choisis dans le groupe des produits d'addition d'oxyde d'éthylène et éventuellement d'oxyde de propylène et d'alcools à longue chaîne ayant de 12 à 18 atomes de carbone dans la chaîne.

5. Préparation d'enzyme selon l'une quelconque des revendications 1 à 4, contenant :
10 à 70 % en poids, de préférence 30 à 50 % en poids, d'un solvant choisi dans le groupe constitué par l'éthylèneglycol, le propylèneglycol, le glycérol et des mélanges de ceux-ci,
0,5 à 5 % en poids, de préférence 1 à 3 % en poids, d'un stabilisant choisi dans le groupe constitué par le polyhexaméthylènebiguanide, la N,N-bis(3-aminopropyl)dodécylamine, leurs sels et des mélanges de ces composés,
1 à 15 % en poids, de préférence 2 à 8 % en poids, d'une enzyme active pour le nettoyage,
10 à 80 % en poids d'autres adjuvants et d'eau.

6. Préparation d'enzyme selon l'une quelconque des revendications 1 à 5, contenant de 5 à 40, en particulier, de 10 à 30 % en poids d'eau.

7. Préparation d'enzyme selon l'une quelconque des revendications 1 à 6, contenant de 5 à 50, en particulier de 20 à 40 % en poids de tensioactifs non ioniques choisis dans le groupe des produits d'addition d'oxyde d'éthylène et éventuellement d'oxyde de propylène avec des alcools gras.

8. Utilisation d'une préparation d'enzyme selon l'une quelconque des revendications 1 à 7, pour la production d'une solution de nettoyage destinée au nettoyage d'appareils de laiterie.

9. Procédé pour le nettoyage d'appareils de laiterie à l'aide d'une solution de nettoyage qui est préparée par dilution d'une préparation d'enzyme selon l'une quelconque des revendications 1 à 7 avec de l'eau dans un rapport allant de 1:5000 à 1:500, de préférence, de 1:3000 à 1:1000, et addition subséquente de 0,01 à 0,2 % en poids, de préférence, de 0,03 à 0,1 % en poids, par rapport à la solution de nettoyage dans son ensemble, d'un concentré tampon pour l'ajustement d'un pH compris entre 7 et 10.

10. Procédé selon la revendication 9, dans lequel le concentré tampon a la composition suivante :
3 à 20 % en poids, de préférence 5 à 10 % en poids, d'un complexant choisi dans le groupe des polyphosphonates, polyacrylates et mélanges de ceux-ci,
3 à 20 % en poids, de préférence 5 à 10 % en poids, d'un hydroxyde de métal alcalin choisi dans le groupe constitué par l'hydroxyde de sodium, l'hydroxyde de potassium et des mélanges de ceux-ci,
5 à 30 % en poids, de préférence 10 à 20 % en poids, d'un carbonate alcalin choisi dans le groupe constitué par le carbonate de sodium, le carbonate de potassium et des mélanges de ceux-ci,
20 à 80 % en poids, de préférence, 40 à 60 % en poids d'eau.

11. Procédé selon la revendication 9 ou 10,
dans lequel
une fois effectué le nettoyage, la solution de nettoyage est récupérée et conservée pour des nettoyages ultérieurs.
